(19) Europäisches Patentamt / European Patent Office / Office européen des brevets

(11) **EP 2 782 546 B1**

(12) **EUROPEAN PATENT SPECIFICATION**

(45) Date of publication and mention
of the grant of the patent:
**18.01.2017 Bulletin 2017/03**

(21) Application number: **12808497.7**

(22) Date of filing: **22.11.2012**

(51) Int Cl.:
*A61K 8/06* (2006.01)  *A61K 8/25* (2006.01)
*A61K 8/31* (2006.01)  *A61K 8/34* (2006.01)
*A61K 8/39* (2006.01)  *A61Q 15/00* (2006.01)

(86) International application number:
**PCT/IB2012/056617**

(87) International publication number:
**WO 2013/076674 (30.05.2013 Gazette 2013/22)**

(54) **LIQUID DEODORANT COMPOSITION OF PICKERING EMULSION TYPE**

PICKERING EMULSION ALS FLÜSSIGE DEODORANTFORMULIERUNG

COMPOSITION DEODORANTE LIQUIDE DE TYPE EMULSION PICKERING

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **25.11.2011 FR 1160793
25.11.2011 FR 1160791
07.12.2011 US 201161567685 P
07.12.2011 US 201161567678 P**

(43) Date of publication of application:
**01.10.2014 Bulletin 2014/40**

(73) Proprietor: **L'Oréal
75008 Paris (FR)**

(72) Inventor: **BARA, Isabelle
94210 La Varenne Saint Hilaire (FR)**

(74) Representative: **Nony
11 rue Saint-Georges
75009 Paris (FR)**

(56) References cited:
**EP-A1- 1 005 849    FR-A1- 2 208 642**

**Description**

The present invention relates to the field of deodorants, and especially to the field of compositions in the form of droplets in suspension in a transparent liquid, which are used after shaking.

The invention relates more particularly to a liquid composition in the form of an oil-in-water emulsion, comprising, in a cosmetically acceptable medium:

(i) a hydrophobic dispersed oily phase comprising at least one apolar hydrocarbon-based oil;
(ii) a continuous aqueous-alcoholic phase comprising at least one $C_1$-$C_4$ monoalcohol;
(iii) at least one deodorant other than a $C_1$-$C_4$ monoalcohol, and
(iv) magnesium silicate particles and/or hydrophobic silica aerogel particles.

The deodorant market, which is nowadays highly developed at the international level, presents every year a very large number of new products.

One of these factors is first the galenical form of the deodorants present on the market. Thus, deodorant compositions in the form of sticks, roll-on applicators, aerosols and creams currently exist.

As regards in particular the sprayable deodorant compositions that are currently on the market, they are especially in the form of aqueous-alcoholic solutions, which have the main advantage of affording a fresh sensation at the time of application to the skin.

However, these aqueous-alcoholic compositions have the drawback of causing stinging and of creating a feeling of dryness when applied to the skin. In order to overcome these drawbacks, it has been proposed in the prior art to enrich the compositions with moisturizers.

There is thus a need for an alcohol-based liquid deodorant aqueous-alcoholic cosmetic composition which has good deodorant efficacy and which does not have the drawbacks described previously.

The object of the present invention is to satisfy this need.

The inventors have demonstrated, unexpectedly, in the context of the present patent application, that the use, as a cosmetic composition, of a composition in the form of an emulsion of oil-in-water Pickering type comprising at least one deodorant, makes it possible to overcome the drawbacks described previously.

Thus, a subject of the present invention is firstly a composition in the form of an oil-in-water emulsion, comprising, in a cosmetically acceptable medium:

(i) a dispersed oily phase comprising at least one apolar hydrocarbon-based oil;
(ii) a continuous aqueous-alcoholic phase comprising at least one $C_1$-$C_4$ monoalcohol;
(iii) at least one deodorant other than a $C_1$-$C_4$ monoalcohol, and
(iv) magnesium silicate particles and/or hydrophobic silica aerogel particles.

The present invention is also directed towards a cosmetic process for treating human body odor, in particular underarm odor, comprising the application to the surface of the skin of a composition in accordance with the invention.

The term "cosmetically acceptable medium" means a medium that is compatible with the skin and/or its integuments or mucous membranes, that has a pleasant color, odor and feel and that does not cause any unacceptable discomfort (stinging, tautness or redness) liable to dissuade the consumer from using this composition.

The compositions in accordance with the invention have the advantage of substantially reducing the tautness and stinging sensations caused by alcohol on the skin. They also provide a softening and nourishing effect to the skin, despite having a high content of alcohol that promotes deodorization of the treated area, especially due to the bactericidal or bacteriostatic activities of the alcohol.

In addition, the compositions in accordance with the invention may have a low viscosity, close to that of water, which enables them to be sprayed.

The use and preparation, in general, of cosmetic compositions of Pickering type free of surfactants has already been proposed, in patent application FR 2 208 642.

However, it is never a question in said document of deodorant compositions.

Patent EP 1 005 849 discloses Pickering-type emulsion compositions for treating the hair or the scalp.

By virtue of its Pickering-type emulsion structure, a composition according to the invention proves to be more particularly appealing, especially when compared with conventional deodorant compositions of aqueous-alcoholic nature.

A composition in accordance with the invention also has the advantage of not requiring the presence of surfactants. The absence of these compounds makes it possible to overcome a certain number of drawbacks, such as those mentioned in patent application FR 2 208 642.

The compositions in accordance with the invention provide, when compared with the usual galenical forms, especially aqueous-alcoholic deodorant solutions, a substantial moisturizing effect and also better sensory effects such as a silkier and less dry feel on the skin.

In certain embodiments, a composition in accordance with the invention may also be colored. In this embodiment, a composition according to the invention may comprise, either in the lipophilic dispersed phase or in the hydrophilic continuous phase, or in each of the two phases, at least one coloring agent.

In the rest of the description, (i) a colorant present in the dispersed lipophilic phase is denoted as a "first colorant" and (ii) a colorant present in the continuous hydrophilic phase is denoted as a "second colorant", including when only one from among the dispersed and continuous phases comprises a colorant.

In other embodiments, a composition according to the invention comprises at least one liposoluble first colorant in the dispersed oily phase (i) and at least one water-soluble second colorant in the continuous aqueous-alcoholic phase (ii).

Advantageously, said first and second colorants give the dispersed and continuous phases of a composition according to the invention shades that differ from each other.

Specifically, the addition of at least one colorant to each of the phases of a composition of Pickering type according to the invention so as advantageously to give these phases different shades makes it possible, after correct shaking, to obtain a third shade that is different from the two preceding ones.

The colorant(s) present, respectively, (i) in the dispersed oily phase and (ii) in the continuous aqueous-alcoholic phase thus constitute homogeneity markers that enable the user to determine simply and directly if the composition according to the invention is sufficiently homogenized to be able to be used.

For the purposes of the invention, two shades are considered as being different from each other when the difference between their respective colors can be distinguished by the user with the naked eye.

In particular, the difference between shades ($\Delta E$) can be measured in the L* to b* colorimetric measuring system as defined according to the CIE 1976 standard.

The value of $\Delta E$ is calculated according to formula (I) below:

$$\triangle E = \sqrt{(L_1 - L_2)^2 + (a_1 - a_2)^2 + (b_1 - b_2)^2},$$

in which:

$L_1$, $a_1$, $b_1$ are the CIE Lab colorimetric space coordinates of the first color to be compared, and
$L_2$, $a_2$, $b_2$ are the CIE Lab colorimetric space coordinates of the second color to be compared.

The fragrancing compositions in accordance with the invention may thus be readily prepared without heating or at room temperature, in contrast with the known techniques for manufacturing emulsions, which are generally prepared with heating. Mention may be made especially of the production of emulsions by simple stirring, for example using a paddle blender.

Preferentially, the composition of the invention will be in liquid form.

For the purposes of the invention, the term "liquid composition" means a composition that is not in solid form and whose viscosity, measured using a Rheomat 180 viscometer at 25°C at a spin speed of 200 rpm after 10 minutes of rotation, is less than or equal to 2 Pa.s and more preferentially ranges from 0.01 Pa.s to 0.5 Pa.s.

**Dispersed oily phase**

As indicated previously, a composition in accordance with the invention comprises a dispersed oily phase comprising at least one apolar hydrocarbon-based oil.

For the purposes of the present invention, the term "apolar oil" means an oil whose solubility parameter at 25°C, $\delta a$, is equal to 0 $(J/cm^3)^{1/2}$.

The definition and calculation of the solubility parameters in the Hansen three-dimensional solubility space are described in the article by C.M. Hansen: "The three dimensional solubility parameters", J. Paint Technol. 39, 105 (1967).

According to this Hansen space:

- $\delta D$ characterizes the London dispersion forces derived from the formation of dipoles induced during molecular impacts;
- $\delta p$ characterizes the Debye interaction forces between permanent dipoles and also the Keesom interaction forces between induced dipoles and permanent dipoles;
- $\delta h$ characterizes the specific interaction forces (such as hydrogen bonding, acid/base, donor/acceptor, etc.); and
- $\delta a$ is determined by the equation: $\delta a = (\delta p^2 + \delta h^2)^{1/2}$.

The parameters $\delta p$, $\delta h$, $\delta D$ and $\delta a$ are expressed in $(J/cm^3)^{1/2}$.

An apolar oil in accordance with the invention is hydrocarbon-based.

The term "hydrocarbon-based oil" means an oil formed essentially from, or even constituted by, carbon and hydrogen atoms, and optionally oxygen and nitrogen atoms, and not containing any silicon or fluorine atoms. It may contain alcohol, ester, ether, carboxylic acid, amine and/or amide groups.

According to one embodiment, the apolar hydrocarbon-based oil according to the invention is free of heteroatoms. The term "heteroatom" means an atom other than carbon or hydrogen.

The apolar oil according to the invention may be nonvolatile.

The term "nonvolatile oil" means any oil whose vapor pressure at room temperature and atmospheric pressure is nonzero and less than 0.02 mmHg and better still less than $10^{-3}$ mmHg.

An apolar hydrocarbon-based oil in accordance with the invention advantageously represents from 5% to 40% by weight relative to the total weight of the composition, preferably from 5% to 30% by weight and preferentially from 10% to 30% by weight relative to the total weight of the composition containing it.

According to one preferred embodiment, the apolar hydrocarbon-based oil used in the present invention is nonvolatile and may be chosen advantageously from linear or branched saturated alkanes.

An apolar hydrocarbon-based oil according to the invention may be chosen from oils whose molecular mass is between 300 and 900 g/mol and preferably between 350 and 800 g/mol.

According to one preferred embodiment, the apolar hydrocarbon-based oil is chosen from linear or branched hydrocarbons of mineral or synthetic origin, preferably from a volatile or nonvolatile liquid paraffin oil, hydrogenated isoparaffin, naphthalene oil, a totally or partially hydrogenated liquid polydecene, isoeicosane, a decene/butene copolymer, or a polybutene/polyisobutene copolymer, and mixtures thereof.

The term "hydrocarbon" means a compound consisting of carbon and hydrogen.

According to one particular embodiment, the following will preferably be used among the apolar hydrocarbon-based oils:

- hydrogenated isoparaffins, for instance the hydrogenated polyisobutene sold under the name Parleam by the company Rossow, or under the name Polysynlane by Nippon Oil & Fat or Polyester Corp.;
- the $C_8$-$C_9$ isoparaffin sold under the name Isopar E by ExxonMobil Chemical;
- the $C_{11}$-$C_{13}$ isoparaffin from Exxon sold under the name Isopar L; or
- the $C_{13}$-$C_{14}$ isoparaffin sold under the name Isopar M by the company ExxonMobil Chemical;
- the liquid paraffins from Petro Canada: Puretol 9, or Blandol from Sonneborn, or Marcol 82 sold by ExxonMobil Chemical, and
- the plant perhydrosqualenes sold under the name Olive Squalane by SOS Corporation Alimentaria, or Vegetable Squalane by Lake Oil, or Exolive by Caroi Line Cosmetica.

A dispersed oily phase according to the invention is in the form of spheres of more or less homogeneous size, which may range between 0.5 and 20 mm, preferably between 0.5 and 10 mm and preferentially between 1 and 5 mm.

The number of oil spheres will vary as a function of the percentage of fragrance, oil and magnesium silicate particles. From 1 to several million per liter of formulation may be counted as a function of their size. These spheres may be present in the upper or lower part of the composition comprising them or in both parts of the composition.

As indicated previously, a dispersed oily phase according to the invention may also comprise at least one first liposoluble coloring agent.

Such a colorant may be of natural or synthetic origin.

For the purposes of the invention, the term "liposoluble colorant" means any generally organic, natural or synthetic compound, which is soluble in an oily phase.

Examples of liposoluble colorants in accordance with the invention that may be mentioned include:

- a violet organic colorant, D&C Violet No. 2 K7014 Chemical name: Alizurol purple SS;
- a green organic colorant, D&C Green No. 6 K7016 / 90097 D&C Green 6 Chemical name: quinizarin green E SS;
- a pink organic colorant, D&C Red No. 21 K7061 / Suncroma D&C Red 21 C 14-032 Chemical name: Eosin; and
- an orange vegetable colorant, Betatene 30% OLV Chemical name: Carotenoids (CI. 75130-E160 A) at 30% in olive oil.

The liposoluble colorants in accordance with the invention may also be chosen from Sudan Red, D&C Red 17, $\beta$-carotene, Sudan Brown, D&C Yellow 11, D&C Orange 5, quinoline yellow and annatto.

The dispersed oily phase according to the invention may also comprise other oils, such as silicone oils or plant oils, provided that they do not impair the stability of the composition according to the invention.

## Continuous aqueous-alcoholic phase

As indicated previously, a composition in accordance with the invention comprises, in addition to a dispersed oily phase, a continuous aqueous phase comprising at least one $C_1$-$C_4$ monoalcohol.

A monoalcohol in accordance with the invention may preferably be chosen from linear $C_1$-$C_4$ hydroxyalkyls. Ethanol will be used more particularly.

Such a monoalcohol is present in a content ranging from 50% to 90% by weight and preferably from 53% to 70% by weight relative to the total weight of the composition.

This continuous aqueous phase comprises at least water. This water is then preferably present in a content ranging from 0.5% to 10% by weight and more preferentially from 1% to 5% by weight relative to the total weight of the composition according to the invention.

As indicated previously, the continuous aqueous-alcoholic phase in accordance with the invention may comprise at least a second liposoluble colorant. This colorant may be of synthetic or plant origin.

Mention may in particular be made, as water-soluble dyes suitable for the invention, of synthetic or natural water-soluble dyes, such as, for example, FDC Red 4, DC Red 6, DC Red 22, DC Red 28, DC Red 30, DC Red 33, DC Orange 4, DC Yellow 5, DC Yellow 6, DC Yellow 8, FDC Green 3, DC Green 5, FDC Blue 1, betanin (beetroot), carmine, copper chlorophyllin, methylene blue, anthocyanins (oenocyanin, black carrot, hibiscus, elder), caramel and riboflavin.

According to one particularly preferred form of the invention, the dispersed oily phase and the continuous aqueous phase are adjusted such that the absolute value of the difference in density ($\Delta d$) between the dispersed oily phase and the continuous aqueous phase is not more than 0.05 and advantageously not more than 0.01.

## Magnesium silicate particles

The composition according to the invention also comprises magnesium silicate particles for stabilizing the composition according to the invention by positioning themselves at the dispersed phase/continuous phase interface.

The magnesium silicates in accordance with the invention may be of natural or synthetic origin.

Talc is particularly preferred as a magnesium silicate in accordance with the invention.

Talcs are hydrated magnesium silicates usually comprising aluminium silicate. The crystal structure of talc consists of repeating layers of a sandwich of brucite between layers of silica.

The talc in accordance with the invention may be chosen more particularly from those sold under the names Rose Talc® and Talc SG-2000® sold by the company Nippon Talc, Luzenac Pharma M® sold by the company Luzenac, J-68BC from US Corporation and Micro ACE-P-3® sold by the company Nippon Talc.

The solid magnesium silicate particles may be used in a content preferably of between 0.05% and 5% by weight, preferentially between 0.1% and 2% by weight and even more preferentially between 0.1% and 1% by weight relative to the total weight of the composition containing them.

They also preferably have a size of between 1 and 30 $\mu$m, preferentially between 1 and 20 $\mu$m and more preferentially between 2 and 15 $\mu$m.

## Hydrophobic silica aerogel particles

A composition according to the invention also comprises silica aerogel particles, which are intended to stabilize the composition according to the invention by positioning themselves at the dispersed phase/continuous phase interface.

Aerogels are ultralight porous materials which were first produced by Kristler in 1932.

They are generally synthesized by a sol-gel process in a liquid medium and then dried by extraction with a supercritical fluid. The supercritical fluid most commonly used is supercritical $CO_2$. This type of drying makes it possible to avoid shrinkage of the pores and of the material.

Other types of drying also make it possible to obtain porous materials starting from gel, namely (i) drying by freeze drying, which consists in solidifying the gel at low temperature and in then subliming the solvent, and (ii) drying by evaporation. The materials thus obtained are referred to respectively as cryogels and xerogels. The sol-gel process and the various drying operations are described in detail in Brinker C.J. and Scherer G.W., Sol-Gel Science, New York, Academic Press, 1990.

The term "hydrophobic silica" means any silica whose surface is treated with silylating agents, for example halogenated silanes such as alkylchlorosilanes, siloxanes, in particular dimethylsiloxanes such as hexamethyldisiloxane, or silazanes, so as to functionalize the OH groups with silyl groups Si-Rn, for example trimethylsilyl groups.

Preferably, the hydrophobic aerogel particles that may be used in the present invention advantageously have a specific surface area per unit of mass (SM) ranging from 500 to 1500 $m^2$/g, preferably from 600 to 1200 $m^2$/g and better still from 600 to 800 $m^2$/g and/or have an oil-absorbing capacity measured at the Wet Point ranging from 5 to 18 ml/g of particles, preferably from 6 to 15 ml/g and better still from 8 to 12 ml/g.

The oil-absorbing capacity measured at the wet point, noted Wp, corresponds to the amount of water that needs to be added to 100 g of particle in order to obtain a homogeneous paste.

It is measured according to the Wet Point method or the method for determining the oil uptake of a powder according to the principle described in standard NF T 30-022. It corresponds to the amount of oil adsorbed onto the available surface of the powder and/or absorbed by the powder by measurement of the wet point, described below:

An amount m = 2 g of powder is placed on a glass plate, and the oil (isononyl isononanoate) is then added dropwise. After addition of 4 to 5 drops of oil to the powder, mixing is carried out using a spatula, and addition of oil is continued until conglomerates of oil and powder have formed. From this point, the oil is added at the rate of one drop at a time and the mixture is subsequently triturated with the spatula. The addition of oil is stopped when a firm, smooth paste is obtained. This paste must be able to be spread over the glass plate without cracks or the formation of lumps. The volume Vs (expressed in ml) of oil used is then noted.

The oil uptake corresponds to the ratio Vs/w.

The aerogel particles of hydrophobic silica used according to the present invention are preferably aerogel particles of silylated silica (INCI name: silica silylate).

The preparation of aerogel particles of hydrophobic silica modified at the surface by silylation is further described in the document US 7 470 725.

Use will be made in particular of hydrophobic silica aerogel particles surface-modified with trimethylsilyl groups, namely trimethylsiloxyl silica particles.

The hydrophobic aerogel particles which can be used in the present invention advantageously exhibit a size, expressed as average diameter (D[0.5]), of less than 1500 $\mu$m and preferably ranging from 1 to 30 $\mu$m, preferably from 5 to 25 $\mu$m, better still from 5 to 20 $\mu$m and even better still from 5 to 15 $\mu$m.

The specific surface per unit of weight can be determined by the nitrogen absorption method, known as the BET (Brunauer-Emmett-Teller) method, described in The Journal of the American Chemical Society, Vol. 60, page 309, February 1938 and corresponding to the international standard ISO 5794/1 (appendix D). The BET specific surface corresponds to the total specific surface of the particles under consideration.

The sizes of the aerogel particles according to the invention can be measured by static light scattering using a commercial particle size analyzer of MasterSizer 2000 type from Malvern. The data are processed on the basis of the Mie scattering theory. This theory, which is exact for isotropic particles, makes it possible to determine, in the case of non-spherical particles, an "effective" particle diameter. This theory is described in particular in the publication by Van de Hulst, H.C., "Light Scattering by Small Particles", Chapters 9 and 10, Wiley, New York, 1957.

According to one advantageous embodiment, the hydrophobic aerogel particles used in the present invention have a specific surface area per unit of mass (SM) ranging from 600 to 800 m$^2$/g and a size, expressed as the mean diameter (D[0.5]), ranging from 5 to 20 $\mu$m and better still from 5 to 15 $\mu$m.

According to one preferred embodiment, VM-2270 will more particularly be used, the particles of which have a mean size ranging from 5 to 15 microns and a specific surface area per unit of the mass ranging from 600 to 800 m$^2$/g.

The hydrophobic aerogel particles used in the present invention may advantageously have a tamped density p ranging from 0.04 g/cm$^3$ to 0.10 g/cm$^3$ and preferably from 0.05 g/cm$^3$ to 0.08 g/cm$^3$.

In the context of the present invention, this density can be assessed according to the following protocol, known as packed density protocol:

40 g of powder are poured into a graduated measuring cylinder and then the measuring cylinder is placed on a Stav 2003 device from Stampf Volumeter. The measuring cylinder is subsequently subjected to a series of 2500 packing actions (this operation is repeated until the difference in volume between two consecutive tests is less than 2%) and then the final volume Vf of packed powder is measured directly on the measuring cylinder.

The tamped density is determined by the ratio: mass m/Vf, in this instance 40/Vf (Vf being expressed in cm$^3$ and m in g).

According to one embodiment, the hydrophobic aerogel particles used in the present invention have a specific surface area per unit of volume SV ranging from 5 to 60 m$^2$/cm$^3$, preferably from 10 to 50 m$^2$/cm$^3$ and better still from 15 to 40 m$^2$/cm$^3$.

The specific surface per unit of volume is given by the relationship:

$$SV = SM * \rho$$

where p is the tamped density expressed in g/cm$^3$ and SM is the specific surface area per unit of mass expressed in m$^2$/g, as defined above.

According to one preferred embodiment, the hydrophobic aerogel particles according to the invention have a specific surface area per unit of mass (SM) ranging from 500 to 1500 m$^2$/g, preferably from 600 to 1200 m$^2$/g and better still from 600 to 800 m$^2$/g, a size expressed as the mean diameter (D[0,5]) ranging from 1 to 30 μm and/or an oil-absorbing capacity measured at the Wet Point ranging from 5 to 18 ml/g of particles, preferably from 6 to 15 ml/g and better still from 8 to 12 ml/g.

As hydrophobic silica aerogels that may be used in the invention, examples that may be mentioned include the aerogel sold under the name VM-2260 (INCI name: Silica silylate), by the company Dow Corning, the particles of which have an average size of about 1000 microns and a specific surface area per unit of mass ranging from 600 to 800 m$^2$/g.

Mention may also be made of the aerogels sold by Cabot under the references Aerogel TLD 201, Aerogel OGD 201 and Aerogel TLD 203, Enova Aerogel MT 1100 and Enova Aerogel MT 1200.

Use will be made more particularly of the aerogel sold under the name VM-2270 (INCI name: Silica silylate), by the company Dow Corning, the particles of which have an average size ranging from 5 to 15 microns and a specific surface area per unit of mass ranging from 600 to 800 m$^2$/g.

The hydrophobic silica aerogel particles may be used in a content preferably of between 0.05% and 5% by weight, preferably more preferentially between 0.1% and 2% by weight and even more preferentially between 0.1% and 1% by weight relative to the total weight of the composition containing them.

## Deodorant

A composition in accordance with the invention comprises at least one deodorant.

The term "deodorant" refers to any substance that is capable of masking, absorbing, improving and/or reducing the unpleasant odor resulting from the decomposition of human sweat by bacteria.

A deodorant according to the invention is different from the alcohols present in the hydrophilic continuous phase as indicated previously.

The deodorants in accordance with the invention may be bacteriostatic agents or bactericidal agents that act on the armpit odor microorganisms, such as 2,4,4'-trichloro-2'-hydroxydiphenyl ether (®Triclosan), 2,4-dichloro-2'-hydroxydiphenyl ether, 3',4',5'-trichlorosalicylanilide, 1-(3',4'-dichlorophenyl)-3-(4'-chlorophenyl)urea (®Triclocarban) or 3,7,11-trimethyldodeca-2,5,10-trienol (®Farnesol).

They may also be chosen from quaternary ammonium salts such as cetyltrimethylammonium salts, cetylpyridinium salts, DPTA (1,3-diaminopropanetetraacetic acid), 1,2-decanediol (Symclariol from the company Symrise), glycerol derivatives, for instance caprylic/capric glycerides (Capmul MCM from Abitec), glyceryl caprylate or caprate (Dermosoft GMCY and Dermosoft GMC, respectively from Straetmans), Polyglyceryl-2 caprate (Dermosoft DGMC from Straetmans), and biguanide derivatives, for instance polyhexamethylene biguanide salts, chlorhexidine and salts thereof, and 4-phenyl-4,4-dimethyl-2-butanol (Symdeo MPP from Symrise).

Among the deodorants in accordance with the invention, mention may also be made of zinc salts, for instance zinc salicylate, zinc gluconate, zinc pidolate; zinc sulfate, zinc chloride, zinc lactate, zinc phenolsulfonate; salicylic acid and derivatives thereof such as 5-n-octanoylsalicylic acid.

The deodorants according to the invention may be odor absorbers such as zinc ricinoleate, sodium bicarbonate; metallic or non-metallic zeolites, cyclodextrins and alum.

They may also be a chelating agent such as Dissolvine GL-47-S from Akzo Nobel, EDTA; DPTA.

It may also be a polyol such as glycerol or propane-1,3-diol (Zemea Propanediol sold by Dupont Tate and Lyle BioProducts), or an enzyme inhibitor such as triethyl citrate.

In the event of incompatibility or to stabilize them, some of the agents mentioned above may be incorporated into spherules, especially ionic or nonionic vesicles, and/or particles (capsules and/or spheres).

The deodorants are present in the compositions according to the invention in a content of between 0.01% and 15% by weight, preferably of between 0.1% and 10% by weight, and preferentially of between 0.2% and 5% relative to the total weight of the composition.

Obviously, the deodorants that may be present in a composition in accordance with the invention must not impair the advantageous properties of the composition indicated previously.

## 1. Cosmetic agents

Besides the presence of at least one deodorant, a composition in accordance with the invention may also contain one or more additional cosmetic agents other than a deodorant.

These cosmetic agents include fragrances and active agents to be applied to the skin.

The cosmetic active agent(s) according to the invention may be chosen from:

- antiwrinkle agents,
- moisturizers,
- free-radical scavengers,
- agents acting on the capillary circulation,
- muscle relaxants,
- antioxidants,
- calmatives, and
- mixtures thereof.

Mention may also be made of emollients or softeners such as sweet almond oil, apricot kernel oil, vitamins, essential fatty acids, insect repellents, propellants, peptizers, UV-screening agents, stabilizers or preserving agents, gellants or thickeners, nacres or glitter flakes, and mixtures thereof.

Obviously, the additional cosmetic agents that may be present in a composition in accordance with the invention must not impair the advantageous properties indicated previously.

These cosmetic agents may be present either in the dispersed phase or in the continuous phase of a composition in accordance with the invention. Thus, the hydrophilic or water-soluble agents will be present in the continuous phase, while the lipophilic or liposoluble agents will be present in the dispersed phase.

## 2. Fragrancing substances

A composition in accordance with the invention may also comprise at least one fragrancing substance, or perfume.

The term "fragrancing substance" means any perfume or aroma capable of giving off a pleasant odor.

Perfumes are compositions especially containing the starting materials described in S. Arctander, Perfume and Flavor Chemicals (Montclair, N.J., 1969), in S. Arctander, Perfume and Flavor Materials of Natural Origin (Elizabeth, N.J., 1960) and in Flavor and Fragrance Materials - 1991, Allured Publishing Co., Wheaton, III.

They may also be natural products, for instance essential oils, absolutes, resinoids, resins, concretes, and/or synthetic products (terpene or sesquiterpene hydrocarbons, alcohols, phenols, aldehydes, ketones, ethers, acids, esters, nitriles or peroxides, which may be saturated or unsaturated, and aliphatic or cyclic).

According to the definition given in international standard ISO 9235 and adopted by the Commission of the European Pharmacopoeia, an essential oil is an odorous product generally of complex composition, obtained from a botanically defined plant raw material, either by steam entrainment, or by dry distillation, or via an appropriate mechanical process without heating (cold pressing). The essential oil is usually separated from the aqueous phase via a physical process that does not result in any significant change in the composition.

Among the essential oils that may be used according to the invention, mention may be made of those obtained from plants belonging to the following botanical families:

Abietaceae or Pinaceae: conifers; Amaryllidaceae; Anacardaceae; Anonaceae: ylang ylang; Apiaceae (for example Umbelliferae): dill, angelica, coriander, sea fennel, carrot, parsley; Araceae; Aristolochiaceae; Asteraceae: yarrow, artemisia, camomile, helichrysum; Betulaceae; Brassicaceae; Burseraceae: frankincense; Carophyllaceae; Canellaceae; Cesalpiniaceae: copaifera (copaiba balsam); Chenopodaceae; Cistaceae: rock rose; Cyperaceae; Dipterocarpaceae; Ericaceae: gaultheria (wintergreen); Euphorbiaceae; Fabaceae; Geraniaceae: geranium; Guttiferae; Hamamelidaceae; Hernandiaceae; Hypericaceae: St-John's wort; Iridaceae; Juglandaceae; Lamiaceae: thyme, oregano, monarda, savory, basil, marjorams, mints, patchouli, lavenders, sages, catnip, rosemary, hyssop, balm; Lauraceae: ravensara, sweet bay, rosewood, cinnamon, litsea; Liliaceae: garlic; Magnoliaceae: magnolia; Malvaceae; Meliaceae; Monimiaceae; Moraceae: hemp, hop; Myricaceae; Myristicaceae: nutmeg; Myrtaceae: eucalyptus, tea tree, paperbark tree, cajuput, backhousia, clove, myrtle; Oleaceae; Piperaceae: pepper; Pittosporaceae; Poaceae: lemon balm, lemongrass, vetiver; Polygonaceae; Renonculaceae; Rosaceae: roses; Rubiaceae; Rutaceae: all citrus plants; Salicaceae; Santalaceae: sandalwood; Saxifragaceae; Schisandraceae; Styracaceae: benjoin; Thymelaceae: agar wood; Tilliaceae; Valerianaceae: valerian, spikenard; Verbenaceae: lantana, verbena; Violaceae; Zingiberaceae: galangal, turmeric, cardamom, ginger; Zygophyllaceae.

Mention may also be made of the essential oils extracted from flowers (lily, lavender, rose, jasmine, ylang ylang, neroli), from stems and leaves (patchouli, geranium, petitgrain), from fruit (coriander, aniseed, cumin, juniper), from fruit peel (bergamot, lemon, orange), from roots (angelica, celery, cardamom, iris, rattan palm, ginger), from wood (pinewood, sandalwood, gaiac wood, rose of cedar, camphor), from grasses and gramineae (tarragon, rosemary, basil, lemongrass, sage, thyme), from needles and branches (spruce, fir, pine, dwarf pine) and from resins and balms (galbanum, elemi, benjoin, myrrh, olibanum, opopanax).

Examples of fragrancing substances are especially: geraniol, geranyl acetate, farnesol, borneol, bornyl acetate, linolool, linalyl acetate, linalyl propionate, linalyl butyrate, tetrahydrolinolool, citronellol, citronellyl acetate, citronellyl formate, citronellyl propionate, dihydromyrcenol, dihydromyrcenyl acetate, tetrahydromyrcenol, terpineol, terpinyl acetate, nopol, nopyl acetate, nerol, neryl acetate, 2-phenylethanol, 2-phenylethyl acetate, benzyl alcohol, benzyl acetate, benzyl salicylate, styrallyl acetate, benzyl benzoate, amyl salicylate, dimethylbenzylcarbinol, trichloromethylphenylcarbinyl acetate, p-tert-butylcyclohexyl acetate, isononyl acetate, vetiveryl acetate, vetiverol, α-hexylcinnamaldehyde, 2-methyl-3-(p-tert-butylphenyl)propanal, 2-methyl-3-(p-isopropylphenyl)propanal, 3-(p-tert-butylphenyl)propanal, 2,4-dimethylcyclohex-3-enylcarboxaldehyde, tricyclodecenyl acetate, tricyclodecenyl propionate, 4-(4-hydroxy-4-methylpentyl)-3-cyclohexenecarboxaldehyde, 4-(4-methyl-3-pentenyl)-3-cyclohexenecarboxaldehyde, 4-acetoxy-3-pentyltetrahydropyran, 3-carboxymethyl-2-pentylcyclopentane, 2-n-4-heptylcyclopentanone, 3-methyl-2-pentyl-2-cyclopentenone, menthone, carvone, tagetone, geranylacetone, n-decanal, n-dodecanal, 9-decen-1-ol, phenoxyethyl isobutyrate, phenylacetaldehyde dimethyl acetal, phenylacetaldehyde diethyl acetal, geranonitrile, citronellonitrile, cedryl acetate, 3-isocamphylcyclohexanol, cedryl methyl ether, isolongifolanone, aubepinonitrile, aubepine, heliotropin, coumarin, eugenol, vanillin, diphenyl ether, citral, citronellal, hydroxycitronellal, damascone, ionones, methylionones, isomethylionones, solanone, irones, cis-3-hexenol and esters thereof, musk-indans, musk-tetralins, musk-isochromans, macrocyclic ketones, musk-macrolactones, aliphatic musks, ethylene brassylate and rose essence, and mixtures thereof.

According to one preferred embodiment of the invention, a mixture of different fragrancing substances that generate in common a note that is pleasant to the user is used.

The fragrancing substances will preferably be chosen such that they produce notes (head, heart and base) in the following families: citrine, aromatic, floral, spicy, woody, gourmand, chypre, fougere, leathery, musk.

The compositions of the invention may thus contain a concentration of less than or equal to 30% by weight of fragrancing substance(s), in particular from 1% to 25% by weight and especially from 3% to 15% by weight of fragrancing substance(s) relative to the total weight of the composition comprising them.

When a composition according to the invention comprises at least 1% by weight of fragrancing substance(s) relative to the total weight of the composition, then the composition also comprises at least one polyalkylene glycol, especially as defined below.

### 3. Polyalkylene glycol

A composition in accordance with the invention may also comprise at least one polyalkylene glycol.

The presence of such a compound in the fragrancing compositions in accordance with the invention advantageously makes it possible to incorporate an amount of greater than or equal to 1% by weight of fragrancing substances into a composition of Pickering type in accordance with the invention, without observing any sedimentation and/or phase separation generally observed when the content of fragrancing substances reaches such proportions in such compositions.

According to one particular embodiment, a polyalkylene glycol in accordance with the invention may be of formula (I) below:

$$H-[O-R-]_n-OH \qquad (I),$$

in which

- R represents a linear alkyl chain containing from 1 to 4 carbon atoms, and
- n is an integer ranging from 4 to 200 and advantageously from 4 to 40.

According to one particular embodiment, n is an integer ranging from 4 to 20 and advantageously from 6 to 10.

According to one particular embodiment, a polyalkylene glycol in accordance with the invention is a polyethylene glycol.

Thus, according to one preferred embodiment, a polyalkylene glycol in accordance with the invention is a polyethylene glycol with n being an integer ranging from 4 to 20 and advantageously from 6 to 10.

According to one preferred embodiment, a polyalkylene glycol in accordance with the invention may be chosen from PEG-8OE such as the product sold under the trade name Polyethylene Glycol 400 DUB PEG-8 from the company Stéarineries Dubois or under the trade name Polyglycol 400 from the company Clariant, or alternatively PEG 6OE sold under the trade name Carbowax PEG-300 from the company Dow chemical.

The polyalkylene glycol(s) are preferably present in the composition in concentrations ranging from 0.1% to 3% and more preferentially from 0.5% to 2% by weight relative to the total weight of the composition.

According to one preferred embodiment, a composition according to the invention comprises at least 1% by weight, relative to the total weight of the composition, of at least one fragrancing substance and at least one polyalkylene glycol, preferably of formula (I) below:

$$H\text{-}[O\text{-}R\text{-}]_n\text{-}OH \qquad (I),$$

in which

- R represents a linear alkyl chain containing from 1 to 4 carbon atoms, and
- n is an integer ranging from 4 to 200, advantageously from 4 to 40, preferably from 4 to 20 and preferentially from 6 to 10.

## 4. Water-soluble metal salt

A composition in accordance with the invention may also comprise at least one water-soluble metal salt.

The term "water-soluble metal salt" means any cosmetic or dermatological metal salt that can be completely dissolved in molecular form in a liquid aqueous phase comprising water or a mixture of water/$C_1$-$C_4$ monoalcohol.

According to the present invention, the term "metal salt" means a salt of a metal, i.e. of a simple substance that is capable of releasing simple cations (Dictionnaire de la Chimie et de ses Applications, Duval & Duval, 3rd Edition, 1978, Technique et Documentation).

The water-soluble metal salts are more particularly chosen from water-soluble salts of alkali metals or of alkaline-earth metals.

As water-soluble salts of alkali metals that are useful according to the invention, mention may be made in particular of sodium or potassium salts.

As water-soluble salts of alkaline-earth metals that are useful according to the invention, mention may be made in particular of magnesium or calcium salts.

These salts may be, for example, carbonates, bicarbonates, sulfates, chlorides, nitrates, acetates or hydroxides, and also salts of $\alpha$-hydroxy acids or salts of fruit acids (citrate, tartrate, lactate or malate), or alternatively salts of amino acids (aspartate, arginate, glucocholate or fumarate).

Preferably, a salt according to the invention is chosen from sodium chloride and calcium chloride. It is preferably calcium chloride.

The water-soluble metal salts in accordance with the invention may be present in a content of between 0% and 5% by weight and preferably between 0.2% and 1% by weight relative to the total weight of the composition.

## Conditioning

A composition according to the invention may constitute a fragrancing composition, a deodorant composition, a composition for caring for and/or treating keratin materials and may especially be in the form of a deodorant product in spray or aerosol form (body mist or body splash), an eau fraiche, an eau de toilette, an eau de parfum or an aftershave lotion.

Preferably, it constitutes a deodorant composition, a haircare composition or a fragrancing composition. A composition in accordance with the invention may, for example, be conditioned in a bottle.

A composition of the invention may be dispensed by means of various systems that are well known to those skilled in the art, such as sprayers with or without pressurized gas, or alternatively roll-on applicators.

A composition according to the invention may be manufactured via the known processes generally used in the field of deodorant, haircare or fragrancing formulations.

The compositions according to the invention may also be applied in the form of fine particles by means of pressurization devices. The devices in accordance with the invention are well known to those skilled in the art and comprise non-aerosol pumps or "atomizers", piezoelectric devices, aerosol containers comprising a propellant and also aerosol pumps using compressed air as propellant. These devices are especially described in patents US 4 077 441 and US 4 850 517 (which form an integral part of the content of the description), more particularly relating to fragrancing compositions.

The compositions conditioned as aerosols in accordance with the invention generally contain conventional propellants, for instance dimethyl ether, isobutane, n-butane, propane or trichlorofluoromethane.

As indicated previously, a composition of Pickering emulsion type in accordance with the invention must be shaken before use so as to make the composition perfectly homogeneous, this being demonstrated by the appearance of a new shade, which is different from those of the dispersed and continuous phases, throughout the composition.

In the description and the examples that follow, unless otherwise mentioned, the percentages are weight percentages and the ranges of values written in the form "between ... and ..." include the stated lower and upper limits. The ingredients are mixed, before being formed, in the order and under conditions that may readily be determined by a person skilled in the art.

The examples below are presented as non-limiting illustrations of the field of the invention.

**EXAMPLES**

**Example 1: Pickering deodorant formulation**

| Compounds | % by weight relative to the total weight of the composition |
|---|---|
| Water | 3 |
| CaCl$_2$ | 0.75 |
| PEG 8OE | 1 |
| Talc | 0.3 |
| Hydrogenated isoparaffin | 25 |
| Fragrance * | 1 |
| Ethylhexyl methoxycinnamate (and) ethylhexyl salicylate (and) butyl methoxydibenzoylmethane (Covabsorb® Sensient) Liposoluble organic UV-screening agent | 1.5 |
| BHT (butylated hydroxytoluene) | 0.04 |
| D&C RED 33 | 0.04 |
| Triclosan | 0.5 |
| Ethanol | qs 100 |
| * the fragrance used comprises 40% hedione, 15% geraniol, 22% linalyl acetate, 2% ethylvanillin, 1% Ambrox DL, 10% rose essence, 5% citral and 5% patchoulol. | |

The difference in density between the two phases of the composition is 0.00635.

The composition thus obtained consists of a two-phase solution containing pink-colored droplets suspended in a perfectly clear liquid.

The droplets are distributed at the top and bottom of the bottle comprising the composition, their size being on average of 1 mm.

After moderate manual shaking for 5 seconds, the composition becomes readily homogenized and may be vaporized uniformly, for example using a standard pump-action bottle.

The result on the skin is a fragranced fluid deposit, which feels fresh on application, and after drying has a silky feel on the skin.

Once the bottle has been left to stand, the original two-phase distribution of the composition is regained after 5 minutes.

After two months, the appearance and the organoleptic qualities of the product are unchanged, both at room temperature and at 37°C.

**Example 2: Comparative deodorant aqueous-alcoholic composition**

| Compounds | % by weight relative to the total weight of the composition |
|---|---|
| Water | qs 100 |
| PEG 8OE | 4 |
| Fragrance * | 1 |

(continued)

| Compounds | % by weight relative to the total weight of the composition |
|---|---|
| Ethylhexyl methoxycinnamate (and) ethylhexyl salicylate (and) butyl methoxydibenzoylmethane (Covabsorb® Sensient) Liposoluble organic UV-screening agent | 1.5 |
| Triclosan | 0.5 |
| BHT (butylated hydroxytoluene) | 0.04 |
| Ethanol | 54.42 |
| * the fragrance used comprises 40% hedione, 15% geraniol, 22% linalyl acetate, 2% ethylvanillin, 1% Ambrox DL, 10% rose essence, 5% citral and 5% patchoulol. | |

The composition thus obtained consists of a sprayable fluid solution for deodorant use.

It gives a fresh sensation on application, but is dry on the skin. In addition, some tautness may be felt. No moisturizing effect is felt, which tends towards a feeling of dryness.

### Example 3: Fragrance-free Pickering deodorant formulation

| Compounds | % by weight relative to the total weight of the composition |
|---|---|
| Water | qs 100 |
| $CaCl_2$ | 0.75 |
| PEG 8OE | 2 |
| Hydrophobic silica aerogel ** | 0.3 |
| Hydrogenated isoparaffin | 20 |
| Triclosan | 0.5 |
| DC Green 5 | 0.04 |
| BHT (butylated hydroxytoluene) | 0.04 |
| ** VM 2270 Aerogel sold by the company Dow Corning. | |

The composition thus obtained consists of a two-phase solution containing green-colored droplets suspended in a perfectly clear liquid.

The result on the skin is a fluid deposit which feels fresh on application and after drying has a silky feel on the skin.

### Claims

1. A composition in the form of an oil-in-water emulsion comprising, in a cosmetically acceptable medium:

(i) a hydrophobic dispersed oily phase comprising at least one apolar hydrocarbon-based oil;
(ii) a continuous aqueous-alcoholic phase comprising at least one $C_1$-$C_4$ monoalcohol present in a content ranging from 50% to 90% by weight relative to the total weight of the composition;
(iii) at least one deodorant active compound other than a $C_1$-$C_4$ monoalcohol, and
(iv) magnesium silicate particles and/or hydrophobic silica aerogel particles,

and when said composition comprises at least 1% by weight of fragrancing substance(s) relative to the total weight of the composition, then said composition also comprises at least one polyalkylene glycol, preferably of formula (I) below:

$$H\text{-}[O\text{-}R\text{-}]_n\text{-}OH \qquad (I),$$

in which

- R represents a linear alkyl chain containing from 1 to 4 carbon atoms, and
- n is an integer ranging from 4 to 200, advantageously from 4 to 40, preferably from 4 to 20 and preferentially from 6 to 10.

2. The composition as claimed in claim 1, in which the hydrophobic aerogel particles have a specific surface area per unit of mass (SM) ranging from 500 to 1500 $m^2/g$, preferably from 600 to 1200 $m^2/g$ and better still from 600 to 800 $m^2/g$, and a size expressed as the mean diameter (D[0.5]) ranging from 1 to 30 $\mu m$ and/or an oil-absorbing capacity measured at the Wet Point ranging from 5 to 18 ml/g of particles, preferably from 6 to 15 ml/g and better still from 8 to 12 ml/g.

3. The composition as claimed in claim 1 or 2, **characterized in that** the hydrophobic silica aerogel particles have a size, expressed as the mean diameter (D[0.5]), ranging from 5 to 25 $\mu m$, better still from 5 to 20 $\mu m$ and even better still from 5 to 15 $\mu m$.

4. The composition as claimed in one of claims 1 to 3, wherein the hydrophobic silica aerogel particles are trimethylsiloxyl silica particles.

5. The composition as claimed in any one of claims 1 to 4, **characterized in that** the hydrophobic silica aerogel particles have a tamped density p ranging from 0.04 $g/cm^3$ to 0.10 $g/cm^3$ and preferably from 0.05 $g/cm^3$ to 0.08 $g/cm^3$.

6. The composition as claimed in any one of claims 1 to 5, **characterized in that** the hydrophobic silica aerogel particles have a specific surface area per unit volume SV ranging from 5 to 60 $m^2/cm^3$, preferably from 10 to 50 $m^2/cm^3$ and better still from 15 to 40 $m^2/cm^3$.

7. The composition as claimed in any one of the preceding claims, **characterized in that** it also comprises at least one fragrancing substance.

8. The composition as claimed in the preceding claim, **characterized in that** said fragrancing substance is present in the composition in a concentration of less than or equal to 30% by weight, preferably ranging from 1% to 25% by weight and more preferentially from 3% to 15% by weight relative to the total weight of the composition.

9. The composition as claimed in any one of the preceding claims, in which the polyalkylene glycol(s) are preferably present in the composition in concentrations ranging from 0.1% to 3% and more preferentially from 0.5% to 2% by weight relative to the total weight of the composition.

10. The composition as claimed in any one of the preceding claims, **characterized in that** it also comprises at least one water-soluble metal salt, preferably chosen from alkali metal salts and alkaline-earth metal salts, especially chosen from sodium chloride and calcium chloride.

11. The composition as claimed in any one of the preceding claims, **characterized in that** it comprises at least one liposoluble first colorant in the dispersed oily phase (i) and at least one water-soluble second colorant in the continuous aqueous-alcoholic phase (ii).

12. The composition as claimed in claim 11, **characterized in that** said first and second colorants give the dispersed and continuous phases of said compositions different shades.

13. The composition as claimed in any one of the preceding claims, **characterized in that** the apolar hydrocarbon-based oil is chosen from linear or branched hydrocarbons of mineral or synthetic origin, and more particularly chosen from a volatile or nonvolatile liquid paraffin oil, hydrogenated isoparaffin, naphthalene oil, a totally or partially hydrogenated liquid polydecene, isoeicosane, a decene/butene copolymer, or a polybutene/polyisobutene copolymer, and mixtures thereof.

14. The composition as claimed in any one of the preceding claims, **characterized in that** said $C_1$-$C_4$ monoalcohol is

chosen from linear $C_1$-$C_4$ hydroxyalkyls, advantageously ethanol.

**15.** The composition as claimed in any one of the preceding claims, **characterized in that** said $C_1$-$C_4$ monoalcohol is present in a content ranging from 53% to 70% by weight relative to the total weight of the composition.

**16.** The composition as claimed in any one of the preceding claims, **characterized in that** the absolute value of the difference in density ($\Delta d$) between the continuous aqueous phase and the dispersed oily phase is not more than 0.05 and advantageously not more than 0.01.

**17.** A cosmetic process for treating human body odor, in particular underarm odor, comprising the application to the surface of the skin of a composition as defined in any one of claims 1 to 16.


**Patentansprüche**

**1.** Zusammensetzung in der Form einer Öl-in-Wasser-Emulsion, umfassend in einem kosmetisch verträglichen Medium:

(i) eine hydrophobe dispergierte Ölphase, umfassend mindestens ein apolares Öl auf Kohlenwasserstoff-Basis;
(ii) eine zusammenhängende geschlossene wässrig-alkoholische Phase, umfassend mindestens einen $C_1$-$C_4$-Monoalkohol, der in einem Gehalt vorhanden ist, welcher in einem Bereich von 50 Gew.-% bis 90 Gew.-%, relativ zum Gesamtgewicht der Zusammensetzung, liegt;
(iii) mindestens eine desodorierende Verbindung, die verschieden von einem $C_1$-$C_4$-Monoalkohol ist, und
(iv) Magnesiumsilicat-Teilchen und/oder hydrophobe Siliciumdioxid-Aerogelteilchen,

und, wenn die Zusammensetzung mindestens 1 Gew.-% an wohlgeruchverleihender Substanz(en), relativ zum Gesamtgewicht der Zusammensetzung, umfasst, dann die Zusammensetzung auch mindestens ein Polyalkylenglycol, bevorzugt gemäß der Formel (I) nachstehend, umfasst:

$$H\text{-}[O\text{-}R\text{-}]_n\text{-}OH \qquad (I),$$

wobei

- R eine lineare Alkylkette, die 1 bis 4 Kohlenstoffatome enthält, darstellt, und
- n eine ganze Zahl in einem Bereich von 4 bis 200, vorteilhafterweise von 4 bis 40, bevorzugt von 4 bis 20 und vorzugsweise von 6 bis 10 ist.

**2.** Zusammensetzung wie in Anspruch 1 beansprucht, wobei die hydrophoben Aerogelteilchen eine spezifische Oberfläche pro Masseeinheit (SM) in einem Bereich von 500 bis 1500 $m^2$/g, bevorzugt von 600 bis 1200 $m^2$/g und noch besser von 600 bis 800 $m^2$/g und eine Größe, ausgedrückt als der mittlere Durchmesser (D[0,5]), in einem Bereich von 1 bis 30 $\mu$m und/oder ein Ölabsorptionsvermögen, gemessen am Nasspunkt, in einem Bereich von 5 bis 18 ml/g Teilchen, bevorzugt von 6 bis 15 ml/g und noch besser von 8 bis 12 ml/g aufweisen.

**3.** Zusammensetzung wie in Anspruch 1 oder 2 beansprucht, **dadurch gekennzeichnet, dass** die hydrophoben Siliciumdioxid-Aerogelteilchen eine Größe, ausgedrückt als der mittlere Durchmesser (D[0,5]), in einem Bereich von 5 bis 25 $\mu$m, noch besser von 5 bis 20 $\mu$m und sogar noch besser von 5 bis 15 $\mu$m aufweisen.

**4.** Zusammensetzung wie in einem der Ansprüche 1 bis 3 beansprucht, wobei die hydrophoben Siliciumdioxid-Aerogelteilchen Trimethylsiloxyl-Siliciumdioxidteilchen sind.

**5.** Zusammensetzung wie in einem der Ansprüche 1 bis 4 beansprucht, **dadurch gekennzeichnet, dass** die hydrophoben Siliciumdioxid-Aerogelteilchen eine Stampfdichte p in einem Bereich von 0,04 $g/cm^3$ bis 0,10 $g/cm^3$ und bevorzugt von 0,05 $g/cm^3$ bis 0,08 $g/cm^3$ aufweisen.

**6.** Zusammensetzung wie in einem der Ansprüche 1 bis 5 beansprucht, **dadurch gekennzeichnet, dass** die hydrophoben Siliciumdioxid-Aerogelteilchen eine spezifische Oberfläche pro Volumeneinheit SV in einem Bereich von 5 bis 60 $m^2/cm^3$, bevorzugt von 10 bis 50 $m^2/cm^3$ und noch besser von 15 bis 40 $m^2/cm^3$ aufweisen.

**7.** Zusammensetzung wie in einem der vorhergehenden Ansprüche beansprucht, **dadurch gekennzeichnet, dass** sie auch mindestens eine wohlgeruchverleihende Substanz umfasst.

**8.** Zusammensetzung wie im vorhergehenden Anspruch beansprucht, **dadurch gekennzeichnet, dass** die wohlgeruchverleihende Substanz in der Zusammensetzung in einer Konzentration von weniger als oder gleich 30 Gew.-%, bevorzugt in einem Bereich von 1 Gew.-% bis 25 Gew.-% und stärker bevorzugt von 3 Gew.-% bis 15 Gew.-%, relativ zum Gesamtgewicht der Zusammensetzung, vorhanden ist.

**9.** Zusammensetzung wie in einem der vorhergehenden Ansprüche beansprucht, wobei das Polyalkylenglycol(e) bevorzugt in der Zusammensetzung in Konzentrationen in einem Bereich von 0,1 % bis 3 % und stärker bevorzugt von 0,5 Gew.-% bis 2 Gew.-%, relativ zum Gesamtgewicht der Zusammensetzung, vorhanden ist.

**10.** Zusammensetzung wie in einem der vorhergehenden Ansprüche beansprucht, **dadurch gekennzeichnet, dass** sie auch mindestens ein wasserlösliches Metallsalz, bevorzugt ausgewählt aus Alkalimetallsalzen und Erdalkalimetallsalzen, insbesondere ausgewählt aus Natriumchlorid und Calciumchlorid, umfasst.

**11.** Zusammensetzung wie in einem der vorhergehenden Ansprüche beansprucht, **dadurch gekennzeichnet, dass** sie mindestens ein fettlösliches erstes farbgebendes Mittel in der dispergierten Ölphase (i) und mindestens ein wasserlösliches zweites farbgebendes Mittel in der zusammenhängenden geschlossenen wässrig-alkoholischen Phase (ii) umfasst.

**12.** Zusammensetzung wie in Anspruch 11 beansprucht, **dadurch gekennzeichnet, dass** die ersten und zweiten farbgebenden Mittel den dispergierten und zusammenhängenden geschlossenen Phasen der Zusammensetzungen unterschiedliche Farbtöne verleihen.

**13.** Zusammensetzung wie in einem der vorhergehenden Ansprüche beansprucht, **dadurch gekennzeichnet, dass** das apolare Öl auf Kohlenwasserstoff-Basis aus linearen oder verzweigten Kohlenwasserstoffen mit Mineral- oder synthetischem Ursprung ausgewählt ist und stärker besonders aus einem flüchtigen oder nicht-flüchtigen flüssigen Paraffinöl, hydriertem Isoparaffin, Naphthalin-Öl, einem vollständig oder partiell hydrierten flüssigen Polydecen, Isoeicosan, einem Decen/Buten-Copolymer oder einem Polybuten/Polyisobuten-Copolymer und Gemischen davon ausgewählt ist.

**14.** Zusammensetzung wie in einem der vorhergehenden Ansprüche beansprucht, **dadurch gekennzeichnet, dass** der $C_1$-$C_4$-Monoalkohol aus linearen $C_1$-$C_4$-Hydroxyalkylen ausgewählt, vorteilhafterweise Ethanol ist.

**15.** Zusammensetzung wie in einem der vorhergehenden Ansprüche beansprucht, **dadurch gekennzeichnet, dass** der $C_1$-$C_4$-Monoalkohol in einem Gehalt in einem Bereich von 53 Gew.-% bis 70 Gew.-%, relativ zum Gesamtgewicht der Zusammensetzung, vorhanden ist.

**16.** Zusammensetzung wie in einem der vorhergehenden Ansprüche beansprucht, **dadurch gekennzeichnet, dass** der absolute Wert des Dichteunterschieds ($\Delta d$) zwischen der zusammenhängenden geschlossenen wässrigen Phase und der dispergierten Ölphase nicht größer als 0,05 und vorteilhafterweise nicht größer als 0,01 ist.

**17.** Kosmetisches Verfahren zum Behandeln von humanem Körpergeruch, insbesondere Achselgeruch, umfassend das Aufbringen einer Zusammensetzung wie in einem der Ansprüche 1 bis 16 definiert auf die Oberfläche der Haut.

**Revendications**

**1.** Composition sous la forme d'une émulsion huile-dans-eau comprenant dans un milieu cosmétiquement acceptable :

(i) une phase huileuse dispersée hydrophobe comprenant au moins une huile hydrocarbonée apolaire;
(ii) une phase hydroalcoolique continue comprenant au moins un mono-alcool en $C_1$-$C_4$ présente en une quantité allant de 50 % à 90 % en poids par rapport au poids total de la composition;
(iii) au moins un agent déodorant différent d'un mono-alcool en $C_1$-$C_4$ et
(iv) des particules de silicate de magnésium et/ou des particules d'aérogel de silice hydrophobe,

et lorsque ladite composition comprend au moins 1 % en poids de substance(s) parfumante(s) par rapport au poids total de la composition, alors ladite composition comprend également comprend également au moins un polyalkylène glycol, de préférence de formule (I) suivante :

$$H-[O-R-]_n-OH \qquad (I),$$

avec

- R représente une chaîne alkyle linéaire ayant de 1 à 4 atomes de carbone, et
- n est un entier allant de 4 à 200, avantageusement de 4 à 40, de préférence de 4 à 20, préférentiellement de 6 à 10.

2. Composition selon la revendication 1, où les particules d'aérogel hydrophobe présentent une surface spécifique par unité de masse (SM) allant de 500 à 1500 $m^2/g$, de préférence de 600 à 1200 $m^2/g$ et mieux de 600 à 800 $m^2/g$, et une taille exprimée en diamètre moyen (D[0,5]) allant de 1 à 30 $\mu$m et/ou une capacité d'absorption d'huile mesurée au WET POINT allant de 5 à 18 ml/g de particules, de préférence de 6 à 15 ml/g et mieux de 8 à 12 ml/g.

3. Composition selon la revendication 1 ou 2, **caractérisée en ce que** les particules d'aérogel de silice hydrophobe présentent une taille exprimée en diamètre moyen (D[0,5]) allant de 5 à 25 $\mu$m, mieux de 5 à 20 $\mu$m et encore mieux de 5 à 15 $\mu$m.

4. Composition selon l'une des revendications 1 à 3, dans laquelle les particules d'aérogel de silice hydrophobe sont des particules de silice triméthylsiloxylée.

5. Composition selon l'une quelconque des revendications 1 à 4, **caractérisée en ce que** les particules d'aérogel de silice hydrophobe présentent une densité tassée p allant de 0,04 $g/cm^3$ à 0,10 $g/cm^3$, de préférence de 0,05 $g/cm^3$ à 0,08 $g/cm^3$.

6. Composition selon l'une quelconque des revendications 1 à 5, **caractérisée en ce que** les particules d'aérogel de silice hydrophobe présentent une surface spécifique par unité de volume SV allant de 5 à 60 $m^2/cm^3$, de préférence de 10 à 50 $m^2/cm^3$ et mieux de 15 à 40 $m^2/cm^3$.

7. Composition selon l'une quelconque des revendications précédentes, **caractérisée en ce qu'**elle comprend en outre au moins une substance parfumante.

8. Composition selon la revendication précédente, **caractérisée en ce que** ladite substance parfumante est présente dans la composition en une concentration inférieure ou égale à 30 % en poids, de préférence allant de 1 % à 25 % en poids et plus préférentiellement de 3 % à 15 % en poids par rapport au poids total de la composition.

9. Composition selon l'une quelconque des revendications précédentes, où le ou les polyalkylèneglycol(s) sont de préférence présents dans la composition dans des concentrations allant de 0,1 % à 3 %, plus préférentiellement de 0,5 % à 2 % en poids par rapport au poids total de la composition.

10. Composition selon l'une quelconque des revendications précédentes, **caractérisée en ce qu'**elle comprend en outre au moins un sel métallique hydrosoluble, de préférence choisi parmi les sels de métaux alcalins et les sels de métaux alcalino-terreux, notamment choisi parmi le chlorure de sodium et le chlorure de calcium.

11. Composition selon l'une quelconque des revendications précédentes, **caractérisée en ce qu'**elle comprend au moins un premier colorant liposoluble dans la phase huileuse dispersée (i) et au moins un deuxième colorant hydrosoluble dans la phase hydroalcoolique continue (ii).

12. Composition selon la revendication 11, **caractérisée en ce que** lesdits premier et second colorants confèrent aux phases dispersée et continue desdites compositions des teintes différentes l'une de l'autre.

13. Composition selon l'une quelconque des revendications précédentes, **caractérisée en ce que** l'huile hydrocarbonée apolaire est choisie parmi les hydrocarbures linéaires ou ramifiés d'origine minérale ou synthétique et plus particulièrement choisie parmi une huile de paraffine liquide, volatile ou non volatile, l'isoparaffine hydrogénée, l'huile de naphtalène, un polydécène liquide totalement ou partiellement hydrogéné, l'isoéicosane, un copolymère décène/butène, ou un copolymère poly-butène/poly-isobutène et leurs mélanges.

**14.** Composition selon l'une quelconque des revendications précédentes, **caractérisée en ce que** ledit mono-alcool en $C_1$-$C_4$ est choisi parmi les hydroxyalkyles linéaires en $C_1$-$C_4$, avantageusement l'éthanol.

**15.** Composition selon l'une quelconque des revendications précédentes, **caractérisée en ce que** ledit mono-alcool en $C_1$-$C_4$ est présent en une quantité allant de 53 % à 70 % en poids par rapport au poids total de la composition.

**16.** Composition selon l'une quelconque des revendications précédentes, **caractérisée en ce que** la valeur absolue de la différence de densité ($\Delta d$) entre la phase aqueuse continue et la phase huileuse dispersée est d'au plus 0,05, avantageusement d'au plus 0,01.

**17.** Procédé de traitement cosmétique des odeurs corporelles humaines, en particulier des odeurs axillaires, comprenant l'application sur la surface de la peau d'une composition telle que définie dans l'une quelconque des revendications 1 à 16.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- FR 2208642
- EP 1005849 A
- US 7470725 B
- US 4077441 A
- US 4850517 A

**Non-patent literature cited in the description**

- *The Journal of the American Chemical Society,* February 1938, vol. 60, 309
- **VAN DE HULST, H.C.** Light Scattering by Small Particles. Wiley, 1957
- **S. ARCTANDER.** Perfume and Flavor Chemicals. Montclair, 1969
- **S. ARCTANDER.** Perfume and Flavor Materials of Natural Origin. Elizabeth, 1960
- Flavor and Fragrance Materials. Allured Publishing Co, 1991